# EUROPÄISCHE PATENTSCHRIFT

(11) **EP 0 864 327 B1**
(45) Veröffentlichungstag und Bekanntmachung des Hinweises auf die Patenterteilung: **06.11.2002**
(21) Anmeldenummer: 98103410.1
(22) Anmeldetag: 27.02.1998
(51) Int. Cl.: A61K 31/505, A61P 35/00

(54) **Verwendung von Pyrimidinderivaten zur Herstellung eines Arzneimittels zur Prävention von Krebs**
Use of pyrimidin derivatives for the manufacture of a medicament to prevent cancer
Utilisation des derivés de pyrimidine pour la fabrication d'un médicament pour la prévention du cancer

(30) Priorität: 13.03.1997 DE 19710435
(43) Veröffentlichungstag der Anmeldung: 16.09.1998
(73) Patentinhaber: HOECHST AKTIENGESELLSCHAFT, 65929 Frankfurt am Main (DE)
(72) Erfinder: Geisen, Karl, Dr., 60318 Frankfurt (DE)

(56) Entgegenhaltungen:
- EP-A- 0 470 616
- US-A- 5 138 058
- K. GEISEN ET AL.: "Sorbitol-accumulating pyrimidine derivatives." ARZNEIMITTELFORSCHUNG, Bd. 44, Nr. 2, 1994, Seiten 1032-1043, XP000652228

## Beschreibung

Die vorliegende Erfindung beschäftigt sich mit der Verwendung von Pyrimidinderivaten zur Herstellung eines Arzneimittels zur Vorbeugung von Krebserkrankungen.

Bei den verwendeten Pyrimidinderivaten handelt es sich um Wirkstoffe der Formel I 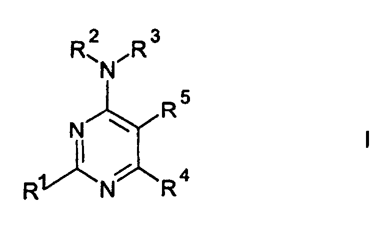 worin bedeuten
- R¹: -CH₂-OH, -CH₃,
- R⁴, R⁵: Wasserstoff,
- R², R³: gemeinsam mit dem Stickstoff, an den sie gebunden sind, eine Piperazinogruppe, wobei diese Piperazinogruppe in 4-Position mit einer N,N-Dimethylaminosulfonylgruppe substituiert ist,
sowie deren physiologisch verträgliche Salze, zur Herstellung eines Arzneimittels zur Vorbeugung der Tumorentstehung.

In US 5,138,058, WO 94/07867 wie auch in der wissenschaftlichen Literatur [z. B. K. Geisen, R. Utz, H. Grötsch, H. J. Lang und H. Nimmesgern, Arzneimittel-Forsch./Drug Res. 44 (II) (1994): 1032 - 1043] werden für die Verbindungen der Formel I eine Vielzahl an pharmakologischen Wirkungen beschrieben. So wird mit den Pyrimidinderivaten der Formel I eine signifikante Verbesserung der Nervenleitgeschwindigkeit erzielt. Außerdem wird bei Behandlung diabetischer Ratten mit den genannten Pyrimidinen eine Normalisierung der glomerulären Filtrationsrate und eine Verminderung der Albuminurie beobachtet. Die in der Literatur beschriebenen Effekte machen die Verbindungen als wertvolle Arzneimittel zur Prophylaxe und Behandlung von Erkrankungen des diabetischen Formenkreises, insbesondere zur Prophylaxe und Behandlung diabetischer Spätschäden geeignet.

Es wurde nun überraschend gefunden, daß die in genannter Literatur und angegebenen Patentschriften beschriebenen Pyrimidinderivate der Formel I in der Lage sind, die Entwicklung von Tumoren zu verringern oder gänzlich zu inhibieren. So sind die genannten Verbindungen bereits allein und ohne Zusatz anderer Stoffe in der Lage, eine günstige therapeutische Hemmung insbesondere der Tumorentstehung zu bewirken.

### Experimenteller Nachweis der Antitumorwirkung

Die tumor-prophylaktische Wirkung der Pyrimidin-Derivate der Formel I wurde an Ratten getestet, die mit Streptozotocin vorbehandelt wurden. Streptozotocin ist ein Methylnitrosohamstoff-Derivat mit alkylierenden Eigenschaften. Es ist eine onkogenund cytotoxisch wirkende Substanz, die von der US Food and Drug Administration zur Behandlung metastatischer Inselzellkarzinome des Pankreas zugelassen wurde. In Ratten führt eine einzige intravenöse Bolusinjektion von Streptozotocin zum akuten Auftreten von Diabetes Mellitus und über einen längeren Zeitraum zur Bildung von Adenomen und Adenokarzinomen der Niere (Lit von Dr. Geisen VII - XII). In diesem Modell streptozotocinbehandelter Ratten führt die chronische Behandlung mit den erfindungsgemäßen Pyrimidinderivaten zur nahezu vollständigen Aufhebung der Entwicklung von Nierentumoren, während 80 % der unbehandelten Tiere die Bildung von Adenokarzinomen in den Nieren zeigen.

### Experimentalbeispiel:

24 männlichen Ratten mit einem Körpergewicht von 210 - 230 g wurden zur Tumorinduktion 60mg/kg Streptozotocin-sulfat intravenös appliziert. Sechs Wochen nach Applikation von Streptozotocin erhielten 12 der 24 diabetischen Tiere mit dem Trinkwasser täglich per oral eine Dosis von 50mg/kg von 2-Methyl-4-(4-N,N-dimethylaminosulfonyl-1-piperazino)pyrimidin zugeführt.

Nach 288 Behandlungstagen wurde der Versuch beendet, wobei 3 Tiere der Kontrollgruppe und 2 Tiere der mit 2-Methyl-4-(4-N,N-dimethylaminosulfonyl-1-piperazino)pyrimidin behandelten Gruppe vorzeitig verstorben waren. Das Nierengewicht der Kontrolltiere war signifikant höher als das Nierengewicht der Tiere, die 2-Methyl-4-(4-N,N-dimethylaminosulfonyl-1-piperazino)pyrimidin erhielten. Nur eines von zehn der mit 2-Methyl-4-(4-N,N-dimethylaminosulfonyl-1-piperazino)pyrimidin behandelten Tiere hatte in einer Niere einen Tumor in der Größe einer Linse entwickelt. Dagegen entwickelten 7 der 9 Kontrolltiere erbsen- bis bohnengroße Tumoren.

Erfindungsgemäß ist deshalb die Verwendung eines Pyrimidinderivates der Formel I zur Herstellung eines Arzneimittels zur Hemmung des Tumorwachstums und zur Vorbeugung der Tumorgenese geeignet.

Bevorzugt ist die Verwendung eines Pyrimidinderivates der Formel I zur Herstellung eines Arzneimittels zur Vorbeugung von Tumorerkrankungen in Kombination mit einem in der Krebsprävention und Krebsbehandlung verwendetem Therapeutikum.

Weiter bevorzugt ist die Verwendung eines Pyrimidinderivates der Formel I zur Herstellung eines Arzneimittels zur Vorbeugung von Tumorerkrankungen in Kombination mit einer physikalischen, tumortherapeutischen Maßnahme, insbesondere einer Strahlentherapie oder einer Hyperthermie-Therapie.

Ebenfalls bevorzugt ist die Verwendung eines Pyrimidinderivates der Formel I zur Herstellung eines Arzneimittels zur Vorbeugung von Tumorerkrankungen in Kombination mit einem Immunmodulator.

Weiterhin bevorzugt ist die Verwendung eines Pyrimidinderivates der Formel I zur Herstellung eines Arzneimittels zur Vorbeugung von Tumorerkrankungen in Kombination mit einem Inhibitor des zellulären Natrium-Wasserstoff-Austauschers.

Besonders bevorzugt ist die Verwendung eines Pyrimidinderivates der Formel I in Kombination mit weiteren Stoffen, die die Wirkung der Pyrimidinderivate verstärken, ohne selbst eine gegen Tumorentstehung und Tumorwachstum gerichtete Wirkung zu besitzen, zur Herstellung eines Arzneimittels zur Vorbeugung von Tumorerkrankungen.

Weiterhin bevorzugt ist die Verwendung eines Pyrimidinderivates der Formel I zur Herstellung eines Arzneimittels zur Vorbeugung von Tumorerkrankungen in Kombination mit pharmakologisch verträglichen Säuren oder säureerzeugenden nutritiven Maßnahmen.

Weiterhin bevorzugt ist die Verwendung eines Pyrimidinderivates der Formel I zur Herstellung eines Arzneimittels zur Vorbeugung von Tumorerkrankungen in Kombination mit Modulatoren der biologischen pH-Regulation.

Weiterhin bevorzugt ist die Verwendung eines Pyrimidinderivates der Formel I zur Herstellung eines Arzneimittels zur Vorbeugung von Tumorerkrankungen in Kombination mit Inhibitoren der Carboanhydratase.

Weiterhin bevorzugt ist die Verwendung eines Pyrimidinderivates der Formel I zur Herstellung eines Arzneimittels zur Vorbeugung von Tumorerkrankungen in Kombination mit einem Inhibitor des Chlorid-Bicarbonat-Exchangers.

Ganz besonders bevorzugt ist die Verwendung von 2-Methyl4-(4-N,N-dimethylaminosulfonyl-1-piperazino)pyrimidin und von 2-Hydroxymethyl-4-(4-N,N-dimethylaminosulfonyl-1-piperazino)pyrimidin als Pyrimidinkomponente eines Tumortherapeutikums.

Die Pyrimidinderivate bewirken bereits allein ohne Zusatz anderer Stoffe eine günstige therapeutische Hemmung des Tumorwachstums bzw. der Tumorentstehung.

Das relativ geringe toxische Potential der hier beschriebenen Pyrimidine kann in vorteilhafter Weise mit anderen in der Krebsbehandlung möglichen, und in vielen Fällen toxischeren Behandlungsformen kombiniert werden, wie z. B.
mit chemotherapeutischen Maßnahmen,
mit Bestrahlungsmaßnahmen,
mit Immunmodulatoren,
mit einer Hyperthermiebehandlung,
mit Inhibitoren des zellulären Natrium-Protonen-Austauschers, wie beispielsweise mit Amilorid oder HOE 642,
mit Stoffen, die über eine inhibitorischen Wirkung auf die Carboanhydratase verfügen, mit paralleler Verabreichung therapeutisch untoxischer und verträglicher Säuren oder säureerzeugende nutritive Behandlung (wie z. B. die Verabreichung größerer Mengen an Glukose/Saccharose, z. B. in Form von Cola).

Der Vorteil einer solchen kombinierten Behandlung kann darin bestehen, daß die derzeit üblichen toxischeren Behandlungsprinzipien (Bestrahlung, Chemotherapie, Hyperthermie) gemildert und vermindert werden können und/oder die tumorhemmende Wirkung eines erfindungsagemäßen Pyrimidinderivates verstärkt werden kann.

## Patentansprüche

1. Verwendung eines Pyrimidinderivates der Formel I 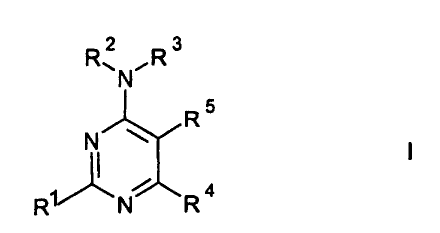 worin bedeuten
R¹ -CH₂OH, -CH₃,
R⁴, R⁵ Wasserstoff,
R², R³ gemeinsam mit dem Stickstoff, an den sie gebunden sind, eine Piperazinogruppe, wobei diese Piperazinogruppe in 4-Position mit einer N,N-Dimethylaminosulfonylgruppe substituiert ist,
sowie dessen physiologisch verträgliche Salze, zur Herstellung eines Arzneimittels zur Vorbeugung der Tumorentstehung.

2. Verwendung von der Kombination enthaltend ein Pyrimidinderivat der Formel I gemäß Anspruch 1 und ein in der Krebsprävention und Krebsbehandlung verwendetem Therapeutikum zur Herstellung eines Arzneimittels zur Vorbeugung von Tumorerkrankungen.

3. Verwendung eines Pyrimidinderivates nach Anspruch 1 zur Herstellung eines Arzneimittels zur Vorbeugung von Tumorerkrankungen in Kombination mit einer physikalischen tumortherapeutischen Maßnahme, insbesondere einer Strahlentherapie oder einer Hyperthermie-Therapie.

4. Verwendung von der Kombination enthaltend ein Pyrimidinderivat der Formel I gemäß Anspruch 1 und einem Immunmodulator zur Herstellung eines Arzneimittels zur Vorbeugung von Tumorerkrankungen.

5. Verwendung von der Kombination enthaltend ein Pyrimidinderivat der Formel I gemäß Anspruch 1 und einem Inhibitor des zellulären Natrium-Wasserstoff-Austauschers zur Herstellung eines Arzneimittels zur Vorbeugung von Tumorerkrankungen.

6. Verwendung von der Kombination enthaltend ein Pyrimidinderivat der Formel I gemäß Anspruch 1 und weitere Stoffe, die die Wirkung der Pyrimidinderivate verstärken, ohne selbst eine gegen Tumorentstehung und Tumorwachstum gerichtete Wirkung zu besitzen, zur Herstellung eines Arzneimittels zur Vorbeugung von Tumorerkrankungen.

7. Verwendung von der Kombination enthaltend ein Pyrimidinderivate der Formel I gemäß Anspruch 1 und pharmakologisch verträglichen Säuren oder säureerzeugenden Stoffen oder Nahrungsmitteln zur Herstellung eines Arzneimittels zur Vorbeugung von Tumorerkrankungen.

8. Verwendung von der Kombination enthaltend ein Pyrimidinderivat der Formel I gemäß Anspruch 1 und Modulatoren der biologischen pH-Regulation zur Herstellung eines Arzneimittels zur Vorbeugung von Tumorerkrankungen.

9. Verwendung von der Kombination enthaltend ein Pyrimidinderivat der Formel I gemäß Anspruch 1 und Inhibitoren der Carboanhydratase zur Herstellung eines Arzneimittels zur Vorbeugung von Tumorerkrankungen.

10. Verwendung von der Kombination enthaltend ein Pyrimidinderivat der Formel I gemäß Anspruch 1 und einem Inhibitor des Chlorid-Bicarbonat-Exchangers zur Herstellung eines Arzneimittels zur Vorbeugung von Tumorerkrankungen.

11. Verwendung gemäß einem der Ansprüche 1 - 8, **dadurch gekennzeichnet, daß** die Verbindung der Formel I 2-Methyl-4-(4-N,N-dimethylaminosulfonyl-1-piperazino)pyrimidin oder 2-Hydroxymethyl-4-(4-N,N-dimethylaminosulfonyl-1-piperazino)pyrimidin ist.

## Claims

1. The use of a pyrimidine derivative of the formula I 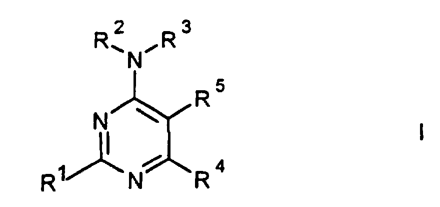 in which
R¹ is -CH₂-OH, -CH₃,
R⁴, R⁵ are hydrogen,
R², R³, together with the nitrogen to which they are bonded, are a piperazino group, where this piperazino group is substituted in the 4-position by an N,N-dimethylaminosulfonyl group,
or its physiologically tolerable salts, for the production of a pharmaceutical for the prevention of tumor formation.

2. The use of the combination comprising a pyrimidine derivative of the formula I as claimed in claim 1 and a therapeutic used in cancer prevention and cancer treatment for the production of a pharmaceutical for the prevention of oncoses.

3. The use of a pyrimidine derivative as claimed in claim 1 for the production of a pharmaceutical for the prevention of oncoses in combination with a physical tumor-therapeutic measure, in particular radiation therapy or hyperthermia therapy.

4. The use of the combination comprising a pyrimidine derivative of the formula I as claimed in claim 1 and an immunomodulator for the production of a pharmaceutical for the prevention of oncoses.

5. The use of the combination comprising a pyrimidine derivative of the formula I as claimed in claim 1 and an inhibitor of the cellular sodium-hydrogen exchanger for the production of a pharmaceutical for the prevention of oncoses.

6. The use of the combination comprising a pyrimidine derivative of the formula I as claimed in claim 1 and other substances which potentiate the action of the pyrimidine derivatives without themselves having an action directed against tumor formation and tumor growth, for the production of a pharmaceutical for the prevention of oncoses.

7. The use of the combination comprising a pyrimidine derivative of the formula I as claimed in claim 1 and pharmacologically tolerable acids or acid-producing substances or foodstuffs for the production of a pharmaceutical for the prevention of oncoses.

8. The use of the combination comprising a pyrimidine derivative of the formula I as claimed in claim 1 and modulators of biological pH regulation for the production of a pharmaceutical for the prevention of oncoses.

9. The use of the combination comprising a pyrimidine derivative of the formula I as claimed in claim 1 and inhibitors of carboanhydratase for the production of a pharmaceutical for the prevention of oncoses.

10. The use of the combination comprising a pyrimidine derivative of the formula I as claimed in claim 1 and an inhibitor of the chloride-bicarbonate exchanger for the production of a pharmaceutical for the prevention of oncoses.

11. The use as claimed in one of claims 1 - 8, wherein the compound of the formula I is 2-methyl-4-(4-N,N-dimethylaminosulfonyl-1-piperazino)pyrimidine or 2-hydroxymethyl-4-(4-N,N-dimethylaminosulfonyl-1-piperazino)pyrimidine.

## Revendications

1. Utilisation d'un dérivé pyrimidique de formule I 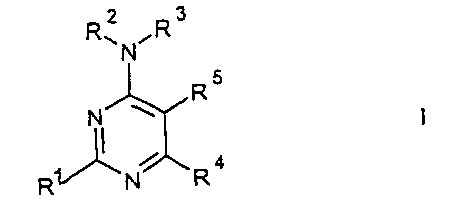 dans lequel représentent
R¹ -CH₂-OH, -CH₃,
R⁴, R⁵ un atome d'hydrogène
R², R³ ensemble avec l'atome d'azote auquel ils sont liés un groupe pipérazino où ce groupe pipérazino est substitué en position 4 avec un groupe N,N-diméthylaminosulfonyle
ainsi que de ses sels physiologiquement tolérants pour la préparation d'un médicament pour la prévention du développement tumoral.

2. Utilisation de la combinaison contenant un dérivé pyrimidique de formule I selon la revendication 1 et un produit thérapeutique utilisé dans la prévention du cancer et le traitement du cancer pour la préparation d'un médicament pour la prévention des maladies tumorales.

3. Utilisation d'un dérivé pyrimidique selon la revendication 1 pour la préparation d'un médicament pour la prévention des maladies tumorales en combinaison avec une mesure de thérapie physique de tumers, en particulier une radiothérapie ou une thérapie par hyperthermie.

4. Utilisation de la combinaison contenant un dérivé pyrimidique de formule I selon la revendication 1 et un d'immunomodulateur pour la préparation d'un médicament pour la prévention des maladies tumorales.

5. Utilisation de la combinaison contenant un dérivé pyrimidique de formule I selon la revendication 1 et un inhibiteur de l'échangeur cellualire sodium-hydrogène pour la préparation d'un médicament pour la prévention des maladies tumorales.

6. Utilisation de la combinaison contenant un dérivé pyrimidique de formule I selon la revendication 1 et d'autres substances qui renforcent l'effet du dérivé pyrimidique sans posséder elles-mêmes un effet contre le développement tumoral et la croissance tumorale, pour la préparation d'un médicament pour la prévention des maladies tumorales.

7. Utilisation de la combinaison contenant un dérivé pyrimidique de formule I selon la revendication 1 et des acides pharmacologiquement tolérants ou des substances dégageant de l'acide ou des aliments pour la préparation d'un médicament pour la prévention des maladies tumorales.

8. Utilisation de la combinaison contenant un dérivé pyrimidique de formule I selon la revendication 1 et des modulateurs de la régulation biologique du pH pour la préparation d'un médicament pour la prévention des maladies tumorales.

9. Utilisation de la combinaison contenant un dérivé pyrimidique de formule I selon la revendication 1 et des inhibiteurs de l'anhydrase carbonique pour la préparation d'un médicament pour la prévention des maladies tumorales.

10. Utilisation de la combinaison contenant un dérivé pyrimidique de formule I selon la revendication 1 et un inhibiteur de l'échangeur chlorure-bicarbonate pour la préparation d'un médicament pour la prévention des maladies tumorales.

11. Utilisation selon une des revendications 1 à 8 **caractérisée en ce que** le composé de formule I est la 2-méthyl-4-(4-N,N-diméthylaminosulfonyl-1-pipérazino)-pyrimidine ou la 2-hydroxyméthyl-4-(4-N,N-diméthylaminosulfonyl-1-pipérazino)-pyrimidine
